(19) Europäisches Patentamt
European Patent Office
Office européen des brevets

(11) EP 0 704 537 A2

(12) **DEMANDE DE BREVET EUROPEEN**

(43) Date de publication:
**03.04.1996 Bulletin 1996/14**

(51) Int Cl.[6]: **C12P 41/00**, C07C 35/00, C11B 9/00, C12P 7/02

(21) Numéro de dépôt: **95401997.2**

(22) Date de dépôt: **01.09.1995**

(84) Etats contractants désignés:
**AT CH DE FR GB IT LI NL**

(30) Priorité: **05.09.1994 FR 9410604**

(71) Demandeur: **RHONE-POULENC CHIMIE**
**F-92408 Courbevoie Cédex (FR)**

(72) Inventeurs:
- **Petre, Dominique**
**F-69006 Lyon (FR)**
- **Didion, Christophe**
**F-88000 Epinal (FR)**

(74) Mandataire: **Dutruc-Rosset, Marie-Claude et al**
**RHONE-POULENC CHIMIE,**
**Direction de la Propriété Industrielle,**
**25, Quai Paul Doumer**
**F-92408 Courbevoie Cédex (FR)**

(54) **Procédé de résolution d'un mélange d'alcools stéréoisomères**

(57) La présente invention a pour objet un procédé de résolution d'un mélange d'alcools stéréoisomères. Elle concerne plus particulièrement un procédé de séparation enzymatique par transestérification de l'un des deux stéréoisomères.

Ledit procédé est caractérisé par le fait qu'il consiste :

- à faire réagir ledit mélange avec un agent d'acylation, en présence d'une hydrolase, en milieu bi-phasique, hydro-organique,
- à séparer l'isomère estérifié de l'isomère non estérifié afin d'obtenir d'une part, l'un des stéréoisomères de l'alcool et d'autre part, l'ester de l'autre stéréoisomère.

EP 0 704 537 A2

## Description

La présente invention a pour objet un procédé de résolution d'un mélange d'alcools stéréoisomères. Elle concerne plus particulièrement un procédé de séparation enzymatique par acylation sélective de l'un des deux stéréoisomères.

La production de composés optiquement actifs purs est un problème qui se pose dans de nombreux domaines techniques tels que par exemple, la pharmacie, l'agrochimie, l'industrie alimentaire (additifs alimentaires, arômes) et également dans l'industrie de la parfumerie.

On s'attend à ce que ce problème prenne une importance croissante car de plus en plus, on constate que dans une application donnée, seul l'un des stéréoisomères présente la propriété recherchée.

Pour résoudre ce problème, on a proposé des méthodes faisant appel à la catalyse enzymatique.

Ainsi, Faber et al ont décrit un procédé de dédoublement d'alcools chiraux, en milieu organique [Synthesis, pp. 895 et suivantes (1992)].

Toutefois, la plupart des procédés décrits sont difficilement transposables à l'échelle industrielle.

Il s'ensuit qu'il est très intéressant de disposer d'un procédé industriel permettant d'accéder au stéréoisomère souhaité.

Il a maintenant été trouvé et c'est ce qui constitue l'objet de la présente invention, un procédé de résolution d'un mélange d'alcools stéréoisomères caractérisé par le fait qu'il consiste :

- à faire réagir ledit mélange avec un agent d'acylation, en présence d'une hydrolase, en milieu bi-phasique, hydro-organique,
- à séparer l'isomère estérifié de l'isomère non estérifié afin d'obtenir d'une part, l'un des stéréoisomères de l'alcool et d'autre part, l'ester de l'autre stéréoisomère.

L'une des caractéristiques du procédé de l'invention est de conduire la réaction de transestérification, en milieu bi-phasique, hydro-organique, hétérogène.

Les avantages obtenus sont ainsi nombreux. L'activité enzymatique est plus grande qu'en milieu organique et les temps de réaction obtenus sont compatibles avec une exploitation industrielle. De plus, en fin de réaction, l'alcool et l'ester obtenus se trouvent en phase organique et l'enzyme en phase aqueuse, ce qui permet de les séparer aisément par simple séparation de phases, et de recycler la phase aqueuse, donc l'enzyme. Dans une variante préférée, l'agent d'acylation se trouve également en phase aqueuse.

Le procédé de l'invention s'applique à tout mélange d'alcools stéréoisomères. Il est de préférence essentiellement soluble en milieu organique et doit être pris en considération par l'enzyme sélectionnée.

Une première classe de substrats auxquels s'applique l'invention, sont les alcools chiraux c'est-à-dire des énantiomères comprenant un atome de carbone asymétrique.

Ils répondent plus particulièrement à la formule (I) suivante :

$$R - OH \qquad (I)$$

dans ladite formule :

- R représente un radical hydrocarboné comprenant au moins 4 atomes, monovalent, substitué ou non, qui peut être un radical aliphatique, acyclique, saturé ou insaturé, linéaire ou ramifié ; un radical carbocyclique ou hétérocyclique, saturé ou insaturé, monocyclique ou polycyclique,
- le groupe hydroxyle pouvant être porté ou non par le carbone asymétrique.

On précisera, sans pour autant limiter la portée de l'invention, que le radical R représente, le reste :

- d'un alcool chiral aliphatique, saturé ou insaturé, linéaire ou ramifié, éventuellement porteur d'un cycle,
- d'un alcool chiral carbocyclique ou hétérocyclique, monocyclique, saturé ou insaturé,
- d'un alcool chiral carbocyclique ou hétérocyclique, polycyclique comprenant au moins deux carbocycles, saturés et/ou insaturés,

L'alcool chiral qui peut intervenir dans le procédé de l'invention répond plus particulièrement, à la formule (I) dans laquelle R représente un radical aliphatique acyclique, saturé ou insaturé, linéaire ou ramifié.

Plus précisément, R représente un radical alkyle, alcényle, alcadiényle, alcynyle, linéaire ou ramifié ayant de préférence de 4 à 40 atomes de carbone.

La chaîne hydrocarbonée peut être éventuellement interrompue par un hétéroatome (par exemple, oxygène ou soufre) ou par l'un des groupes suivants : -CO-, -COO-, OCOO-, $-SO_2-$ ,

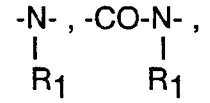

et/ou porteuse de l'un des substituants suivants : -OH, $-COOR_1$, -CHO, $-NO_2$, -X, $-CF_3$ : dans ces formules $R_1$ représente de préférence l'hydrogène ou un radical alkyle linéaire ou ramifié ayant de 1 à 4 atomes de carbone, de préférence, un radical méthyle ou éthyle.

Ledit radical aliphatique acyclique, saturé ou insaturé, linéaire ou ramifié peut être éventuellement porteur d'un substituant cyclique.

Il peut être relié au cycle par un lien valentiel ou par l'un des groupes suivants : -O-, -CO-, -COO-, -OCOO-, -S-, $-SO_2-$ ,

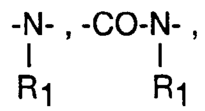

dans ces formules, $R_1$ ayant la signification donnée précédemment.

Par cycle, on entend un cycle carbocyclique ou hétérocyclique, saturé, insaturé ou aromatique.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs d'un ou plusieurs substituants. Les substituants cycliques préférés sont les noyaux benzéniques.

Dans les alcools répondant à la formule (I), R peut représenter un radical carbocyclique, monocyclique. Le nombre d'atomes de carbone dans le cycle peut varier largement de 3 à 8 atomes de carbone mais il est de préférence égal à 5 ou 6 atomes de carbone.

Le carbocycle peut être saturé ou comprenant 1 ou 2 insaturations dans le cycle, de préférence de 1 à 2 doubles liaisons.

Dans le cas où R représente un radical carbocyclique, monocyclique, saturé ou insaturé, il est possible que l'un ou plusieurs des atomes du carbone du cycle soient remplacés par un hétéroatome, de préférence, oxygène, azote ou soufre ou par un groupe fonctionnel, de préférence carbonyle ou ester, conduisant ainsi à un composé hétérocyclique, monocylique. Le nombre d'atomes dans le cycle peut varier largement de 3 à 8 atomes mais il est de préférence égal à 5 ou 6 atomes.

Le radical R peut être également carbocyclique, polycyclique, de préférence bicyclique ce qui signifie qu'au moins deux cycles ont deux atomes de carbone en commun. Dans le cas des radicaux polycycliques, le nombre d'atomes de carbone dans chaque cycle varie entre 3 et 6 : le nombre total d'atomes de carbone étant égal de préférence à 7.

On donne ci-après des exemples de structure bicyclique, couramment rencontrée :

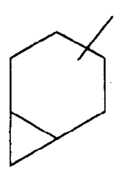
[4,1,0]

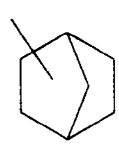
[2,2,1]

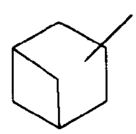
[3,1,1]

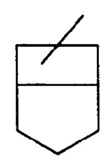
[3,2,0]

Le radical R peut être également hérérocyclique, polycyclique, de préférence bicyclique ce qui signifie qu'au moins deux cycles ont deux atomes en commun. Dans ce cas, le nombre d'atomes dans chaque cycle varie entre 3 et 6 et est plus préférentiellement égal à 5 ou 6.

Il est à noter que si le radical R comprend un cycle quelconque, il est possible que ce cycle porte un substituant. La nature du substituant est quelconque dans la mesure où il n'interfère pas au niveau du produit désiré. Les substituants portés le plus souvent par le cycle sont un ou plusieurs radicaux alkyle ou alkoxy ayant de préférence de 1 à 4 atomes de carbone, de préférence trois radicaux méthyle, un radical méthylène (correspondant à une liaison exocyclique), un radical alcényle, de préférence un radical isopropène-yle, un atome d'halogène, de préférence chlore ou brome.

Les substrats mis en oeuvre préférentiellement dans le procédé de l'invention sont les alcools aliphatiques, saturés ou insaturés, linéaires ou ramifiés, ayant de 4 à 30 atomes de carbone, pouvant éventuellement portés un substituant, par exemple, cyclique, et plus particulièrement, un groupe phényle, un atome d'halogène, un groupement amine ou alkoxy ayant généralement de 1 à 4 atomes de carbone.

Comme exemples plus spécifiques, de mélanges d'alcools stéréoisomères qui peuvent être mis en oeuvre dans le

procédé de l'invention, on peut mentionner, entre autres, le mélange de stéréoisomères du 2-phényl 1-propanol (ou alcool hydratropique), du 1-phényléthanol (ou alcool $\alpha$-phényléthylique), du menthol ou du nortricyclanol.

Une autre catégorie de substrats susceptibles d'être mis en oeuvre dans le procédé de l'invention, sont les stéréoisomères de position, notamment les stéréoisomères d'alcools cycliques secondaires, de préférence du cyclohexanol, l'un présentant le groupe hydroxyle en position axiale et l'autre en position équatoriale. Dans lesdits stéréoisomères, la conformation est bloquée par la présence d'un groupement encombrant, de préférence un groupe alkyle aliphatique ou cycloaliphatique, ayant au moins 3 atomes de carbone jusqu'à 12 atomes de carbone.

Les exemples d'alcools suivants illustrent tout à fait ce type de mélanges de stéréoisomères : le 4-tert-butylcyclohexanol, le 4-isobornylcyclohexanol, le 4-isocamphylcyclohexanol.

Il est à noter que le rapport entre les deux stéréoisomères est tout à fait quelconque. Il peut varier par exemple entre 0,3 et 1,0.

Pour ce qui est du choix de l'agent d'acylation, il est déterminé de telle sorte qu'il soit préférentiellement soluble en phase aqueuse.

On fait appel de préférence, à un ester d'acide organique ou à un anhydride d'acide organique.

Il répond plus particulièrement à la formule (II) :

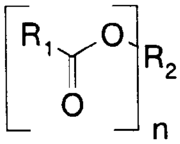

(II)

dans laquelle :

- n est un nombre égal à 1, 2 ou 3,
- $R_1$ représente un radical alkyle ayant une faible condensation en carbone,
- $R_2$ peut prendre les significations suivantes :
  - . si n = 1, $R_2$ représente un radical alkyle ou alcényle de faible condensation en carbone ou un radical -CO-$R_3$, avec $R_3$, identique ou différent de $R_1$, ayant la même signification que $R_1$,
  - . si n = 2, $R_2$ représente un radical alkylène ou alcénylène de faible condensation en carbone,
  - . si n = 3, $R_2$ représente un radical alkyle triyle de faible condensation en carbone.

Dans le présent texte, on entend par l'expression "faible condensation en carbone", moins de 4 atomes de carbone, de préférence, moins de 3 atomes de carbone.

$R_1$ représente préférentiellement, un radical alkyle ayant de 1 à 3 atomes de carbone, tel que méthyle, éthyle, propyle, isopropyle, de préférence un radical méthyle.

Lorsque l'agent d'acylation est un ester d'acide organique, les composés préférés répondent à la formule (II) dans laquelle $R_2$ représente : un radical méthyle ou vinyle, halogénométhyle, mono-, di- ou trihalogénométhyle, méthylsulfate, éthylsulfate pour n = 1 ; un radical diéthylène pour n = 2; un radical propanetriyle-1,2,3 pour n = 3.

Lorsque l'agent d'acylation est un anhydride d'acide, les composés préférés répondent à la formule (II) dans laquelle $R_1$ et $R_3$ sont identiques et représentent un radical alkyle ayant de 1 à 3 atomes de carbone.

A titre illustratif d'agents d'acylation, on peut citer plus particulièrement :

- la triacétine,
- l'acétate de vinyle,
- l'anhydride acétique.

L'agent d'acylation préférentiellement mis en oeuvre dans la procédé de l'invention est la triacétine.

La réaction de transestérification est conduite en présence d'un catalyseur qui est une enzyme choisie parmi les hydrolases, et plus particulièrement les lipases, estérases ou acylases.

Pour la définition des enzymes, on fait référence à la nomenclature internationale définie dans "Enzyme Nomenclature 1984, IUB, Academic Press Inc. (1984). "

Conformément à la présente invention, on fait appel, plus précisément, à une hydrolase appartenant à la classe EC.3 selon la nomenclature précitée (pp. 270) et plus particulièrement aux carboxylestérases (3.1.1.1), aux triacylglycérol lipases (3.1.1.3) ou aux acylases (3.5.1.4).

Tous ces types d'enzymes sont disponibles dans le commerce.

On donne ci-après des exemples d'enzymes convenant tout à fait bien à la mise en oeuvre du procédé de l'invention.

Les lipases sont issues d'organes (par exemple foies, pancréas ou glandes salivaires) d'organismes supérieurs comme de bovins (veaux), porcs, chevaux, lapins ou proviennent de micro-organismes tels que, par exemple : *Géotricum candidum, Penicillium cyclopium, Penicillium roqueforti, Candida cylindracea*, *Rhizopus delemar, Rhizopus niveus, Mucor javanicus, Pseudomonas fluorescences, Pseudomonas species*.

Les estérases peuvent être en particulier des estérases de foie de porc ou de cheval et les acylases obtenues à partir des micro-organismes tels que *l'Aspergillus*.

L'enzyme mise en oeuvre préférentiellement dans le procédé de l'invention est obtenue à partir du *Candida cylindracea.*

Les enzymes mises en oeuvre peuvent être libres ou supportées.

L'enzyme doit être capable de discriminer l'un des deux stéréoisomères, afin d'en estérifier qu'un seul, majoritairement.

Afin de savoir si l'enzyme est bien adaptée à la séparation d'énantiomères à faire, il y a lieu de déterminer le ratio énantiomérique E et le choisir de telle sorte qu'il soit supérieur à 5, de préférence supérieur à 10.

Le ratio énantiomérique E est défini par le rapport suivant :

$$E = \frac{\ln\ [1 - TT\ (\ 1 + ee_p)]}{\ln\ [1 - TT\ (\ 1 - ee_p)]}$$

dans lequel TT représente le taux de transformation du substrat (l'un des stéréoisomères) et $ee_p$ l'excès énantiomérique du produit obtenu.

Comme mentionné précédemment, l'une des caractéristiques du procédé de l'invention est de conduire la réaction en milieu bi-phasique comprenant donc une phase aqueuse et une phase organique constituée par au moins un solvant organique non miscible à l'eau.

Le pH de la phase aqueuse est choisi de telle sorte qu'il soit compatible avec l'activité de l'enzyme. Généralement, ce pH se situe entre 5 et 8 et l'on préfère se placer entre 5 et 7.

Il peut s'avérer nécessaire d'additionner un agent de neutralisation, le plus souvent la soude.

La quantité ajoutée sera telle que l'on obtienne le pH souhaité.

Il est également possible de contrôler le maintien du pH, par ajout d'un agent tampon tel que par exemple, phosphate, carbonate, bicarbonate, borate voire-même un agent tampon organique comme le tris-(hydroxyméthyl)amino-méthane.

En ce qui concerne le solvant organique, on fait appel à un solvant organique non miscible à l'eau.

Plusieurs impératifs président au choix du solvant.

Le solvant doit permettre la solubilisation de toute ou partie du mélange d'alcools. On préfère que les stéréoisomères soient complètement solubilisés.

Une autre condition est que le solvant organique soit inerte vis-à-vis de la réaction de transestérification.

Une classe de solvants préférés sont les solvants organiques, aprotiques, peu polaires.

A titre d'exemples non limitatifs de solvants convenant dans le procédé de l'invention, on peut citer :

- les hydrocarbures aliphatiques et plus particulièrement les paraffines tels que notamment, le pentane, l'hexane, l'heptane, l'octane, l'isooctane, le nonane, le décane, l'undécane, le tétradécane, l'éther de pétrole et le cyclohexane ; les hydrocarbures aromatiques comme notamment le benzène, le toluène, les xylènes, l'éthylbenzène, les diéthylbenzènes, les triméthylbenzènes, le cumène, le pseudocumène, les coupes pétrolières constituées de mélange d'alkylbenzènes notamment les coupes de type Solvesso®,

- les hydrocarbures halogénés aliphatiques ou aromatiques, et l'on peut mentionner : les hydrocarbures perchlorés tels que notamment le trichlorométhane, le tétrachlorure de carbone, le tétrachloroéthylène, l'hexachloroéthane ; les hydrocarbures partiellement chlorés tels que le dichlorométhane, le dichloroéthane, le tétrachloroéthane, le trichloroéthylène, le 1-chlorobutane, le 1,2-dichlorobutane ; le monochlorobenzène, le 1,2-dichlorobenzène, le 1,3-dichlorobenzène, le 1,4-dichlorobenzène ou des mélanges de différents chlorobenzènes,

- les éther-oxydes aliphatiques, cycloaliphatiques ou aromatiques et, plus particulièrement, le diéthyléther, le dipropyléther, le diisopropyléther, le dibutyléther, le méthyltertiobutyléther, le ditertiobutyléther, le diméthyléther de l'éthylèneglycol, le diméthyléther du diéthylèneglycol ; le diphényléther, le dibenzyléther, l'anisole, le phénétole, le 1,4-diméthoxybenzène, le vératrole ; le 1,4-dioxane, le tétrahydrofurane (THF).

Parmi les solvants précités, on choisit préférentiellement les hydrocarbures aliphatiques et aromatiques, de préférence, le pentane, l'heptane, l'éther de pétrole, le toluène.

La quantité de solvant organique mise en jeu peut être très variable. Le rapport volumique entre la phase aqueuse et la phase organique peut varier entre 0,1 et 10, de préférence entre 1,0 et 5,0. Un mode préféré consiste à choisir un rapport volumique supérieur ou égal à 1,0.

La quantité d'eau mise en jeu est la quantité d'eau nécessaire pour que l'on se situe au-delà de la solubilité de l'eau dans le solvant organique considéré : la solubilité étant définie par des tables dans la littérature. La quantité d'eau est de préférence en excès par rapport à cette quantité, d'au moins 100 %, voire des excès nettement supérieurs.

La concentration du mélange d'alcools stéréoisomères dans le solvant organique est fonction de sa solubilité. On choisit, avantageusement une concentration élevée, de l'ordre de 1 mole/litre.

La quantité de l'agent d'acylation à mettre en oeuvre exprimée par rapport à la quantité de stéréoisomère de l'alcool à transformer, est de préférence au moins égale à la quantité stoechiométrique. D'une manière préférentielle, on l'introduit en une quantité supérieure à la quantité stoechiométrique : l'excès pouvant varier de 1 à 40 par rapport à la stoechiométrie, de préférence de 1 à 5.

Pour ce qui est de la quantité d'enzyme mise en oeuvre, on utilise de 0,1 à 100 %, de préférence de 1 à 10 % en poids d'enzyme par rapport au mélange d'alcools stéréoisomères.

La température de la réaction doit être compatible avec l'activité de l'enzyme. Elle est choisie de préférence inférieure à 60°C, et plus préférentiellement entre 20 et 40°C.

La réaction est conduite avantageusement sous pression atmosphérique mais il n'y a aucun inconvénient à utiliser des pressions inférieures ou supérieures à la pression atmosphérique.

D'un point de vue pratique, le procédé selon l'invention est simple à mettre en oeuvre.

On donne ci-après un mode de réalisation préférentielle de la présente invention.

On commence à préparer séparément les phases aqueuse et organique.

La phase aqueuse est constituée du mélange d'eau, de l'enzyme et éventuellement l'agent permettant d'obtenir le bon pH.

Par ailleurs, on introduit le mélange d'alcools stéréoisomères à séparer dans le solvant organique ce qui compose la phase organique.

On procède au mélange des phases aqueuse et organique puis l'on initie la réaction par ajout de l'agent d'acylation.

On porte le milieu réactionnel à la température désirée, tout en maintenant le milieu réactionnel sous bonne agitation.

La durée de la réaction est fonction du taux de transformation de l'alcool à estérifier. Dans le cas où la sélectivité de l'enzyme n'est pas très bonne, on a recours à une technique connue de l'Homme du métier qui est de limiter le taux de transformation.

En fin de réaction, on obtient dans la phase organique, l'un des stéréoisomères sous forme estérifiée et également l'autre stéréoisomère qui reste sous forme d'alcool.

On sépare les phases organique et aqueuse.

On traite la phase organique afin d'éliminer le solvant organique par distillation.

On sépare l'ester obtenu de l'alcool selon les techniques classiques de séparation, de préférence la distillation ou la cristallisation.

Afin d'obtenir l'autre isomère, il est à noter que l'ester peut être rétrogradé en alcool selon les techniques habituelles telles que l'hydrolyse en milieu acide (acide sulfurique, acide trifluorométhanesulfonique).

La phase aqueuse qui comprend l'enzyme peut être avantageusement recyclée.

Il est à noter qu'il est également possible d'acyler l'alcool stéréoisomère obtenu, et en particulier d'effectuer son acétylation, selon les techniques classiques décrites dans la littérature, [Jerry MARCH, Advanced Organic Chemistry, 4ème édition, John Wiley and Sons, 1992, pp. 491], notamment par acétylation à l'aide du chlorure d'acétyle ou d'anhydride acétique.

Le procédé de l'invention convient parfaitement bien à la résolution de nombreux mélanges d'alcools stéréoisomères, notamment celui du 4-tert-butylcyclohexanol constitué de deux stéréoisomères se distinguant par la présence d'un groupe hydroxyle, en position axiale ou équatoriale.

Une variante préférée consiste à transestérifier ledit mélange à l'aide de triacétine ce qui conduit à la séparation des deux stéréoisomères selon les réactions suivantes :

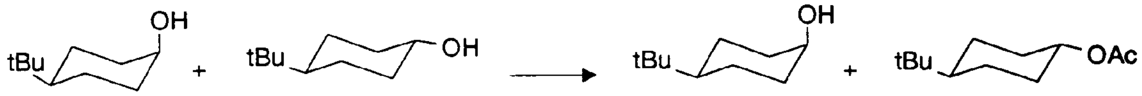

On peut ainsi selon l'invention séparer l'isomère présentant un groupe OH en position axiale par rapport à celui présentant un groupe OH en position équatoriale.

Le procédé de l'invention conduit à des alcools stéréoisomères séparés ou à des alcools acylés, de préférence, acétylés stéréoisomères séparés.

Lesdits produits trouvent de nombreuses applications notamment dans le domaine de la pharmacie.

Un domaine d'application privilégié desdits produits est le domaine de la parfumerie.

Ils peuvent être utilisés, comme ingrédients parfumants, dans les compositions parfumantes, substances et produits parfumés.

Par "compositions parfumantes", on désigne des mélanges de divers ingrédients tels que solvants, supports solides ou liquides, fixateurs, composés odorants divers, etc..., dans lesquels sont incorporés les alcools stéréoisomères ou les alcools acylés stéréoisomères, lesquels sont utilisés pour procurer à divers types de produits finis, la fragrance recherchée.

Les bases pour parfum constituent des exemples préférés de compositions parfumantes dans lesquelles les produits de l'invention peuvent être avantageusement utilisés.

Les eaux de toilettes, les lotions après rasage, les parfums, les savons, les gels de bain ou de douche ou les produits déodorants ou antiperspirants, qu'ils soient sous forme de sticks ou de lotions constituent des exemples de substances ou de produits finis dans lesquels les produits de l'invention apportent leur note originale.

Ils peuvent intervenir aussi dans les shampooings et dans les produits capillaires de tout type.

Ils peuvent aussi parfumer les talcs ou poudres de toute nature.

Ils peuvent également convenir pour les désodorisants d'air ambiant ou tout produit d'entretien.

Un autre exemple de compositions dans lesquelles lesdits composés peuvent être introduits de façon avantageuse, est représenté par les compositions détergentes usuelles. Ces compositions comprennent généralement un ou plusieurs des ingrédients suivants : agents tensio-actifs anioniques, cationiques ou amphotères, agents de blanchiment, azurants optiques, charges diverses, agents anti-redéposition. La nature de ces divers composants n'est pas critique et les produits de l'invention peuvent être ajoutés à tout type de composition détergente. Ils peuvent être introduits dans les adoucissants pour textiles, sous forme liquide ou dans les compositions déposées sur support, le plus souvent un non-tissé, destinées à être employées dans les sèche-linges.

La teneur des compositions selon l'invention en produits de l'invention exprimée en pourcentage en poids dans la composition considérée dépend de la nature de ladite composition (base pour parfum ou eau de toilette par exemple) et de la puissance et de la nature de l'effet recherché au niveau produit final. Il va de soi que dans une base pour parfum la teneur en produit de l'invention peut être très importante, par exemple supérieure à 50 % en poids et peut atteindre 90 % en poids tandis que dans un parfum, une eau de toilette ou une lotion après rasage, cette teneur pourra être très inférieure à 50 % en poids.

La teneur en produit de l'invention peut être dans les compositions détergentes, notamment ménagères ou dans des savons, de l'ordre de 1 à 2%.

Il peut également intervenir dans les shampooings parfumés à raison de 0,5 à 2 % ou pour parfumer tout produit capillaire.

Ainsi la limite inférieure de la teneur en produits de l'invention peut être celle qui provoque une modification perceptible à l'odorat de la fragrance ou de la note du produit fini. Dans certains cas, cette teneur minimale peut être de l'ordre de 0,01 % en poids. On peut évidemment faire appel à des teneurs non comprises dans les limites des teneurs indiquées ci-avant sans pour autant sortir du cadre de la présente invention.

Parmi tous les alcools stéréoisomères et les alcools acylés stéréoisomères obtenus selon le procédé de l'invention, il est à noter que conviennent bien à une application dans le domaine de la parfumerie, les stéréoisomères de position d'alcools ou d'alcools acylés cycliques secondaires, de préférence du cyclohexanol, l'un des stéréoisomères présentant un groupe hydroxyle ou ester (de préférence, acétate) en position axiale et l'autre, un groupe hydroxyle ou ester (de préférence, acétate), en position équatoriale : la conformation étant bloquée par la présence d'un groupement encombrant, qui est de préférence, un groupe tert-butyle, isobornyle ou isocamphyle.

Un composé préféré est l'acétate de 4-tert-butylcyclohexyle axial, qui présente une odeur pin fleuri intéressante.

On donne ci-après des exemples de réalisation pratique de l'invention.

Les exemples qui suivent, illustrent l'invention sans toutefois la limiter.

$$TT = \frac{\text{nb de moles d'alcools stéréoisomères transformées}}{\text{nb de moles d'alcools stéréoisomères engagées}}$$

$$RT_{\text{agent d'acylation}} = \frac{\text{nb de moles d'ester formées}}{\text{nb de moles d'agent d'acylation transformées}}$$

$$RR_{\text{alcool}} = \frac{\text{nb de moles d'ester formées}}{\text{nb de moles d'alcool engagées}}$$

$$\text{Excès énantiomérique ee} = \frac{|\text{nb de moles de } (S) - \text{nb de moles de } (R)|}{\text{nb de moles de } (S) + \text{nb de moles de } (R)}$$

Excès diastéréoismèrique ed =

$$ed = \frac{|\text{nb de moles d'isomère équatorial - nb de moles d' isomère axial}|}{\text{nb de moles d'isomère équatorial + nb de moles d' isomère axial}}$$

Activité = quantité d'alcool transformé en g, par heure et par kg d'enzyme.

EXEMPLES :

Exemple 1 :

. Résolution du 1-phényléthanol

Le mélange est résolu par acétylation de la forme (R) selon la réaction suivante :

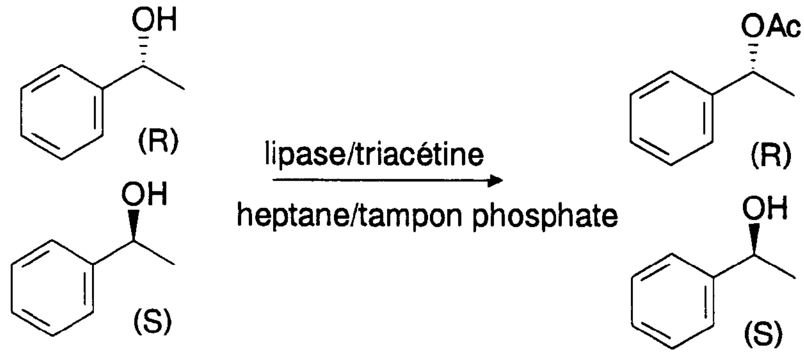


Dans un tube en verre de 4 ml, on charge 10 mg de lipase PS (provenant de *Pseudomonas fluorescences*), commercialisée par la Société AMANO, et 0,4 ml d'un tampon phosphate de sodium de pH égal à 7 (100 mmol/l).

On additionne 10 mg de 1-phényléthanol (0,082 mmol) dissous dans 0,5 ml d'heptane.

On mélange la phase aqueuse et la phase organique, par agitation magnétique.

On ajoute alors 100 µl (0,5 mmol) de triacétine.

Après 60 minutes d'agitation à la température ambiante, on détermine le taux de transformation par chromatographie en phase gazeuse (colonne CARBOWAX® de 15 m) et les excès énantiomériques par chromatographie liquide en phase chirale (colonne CHIRALCEL OJ®).

Les résultats obtenus sont les suivants :

TT = 19 %
ee [1-phényléthanol ] = 24 %
ee [1-acétoxy 1-phényléthane ] = 100 %
E > 100
activité = 190 g/h.kg d'enzyme

Exemple 2 :

Résolution de l'alcool hydratropique

Le mélange est résolu par acétylation de la forme (S) selon la réaction suivante :

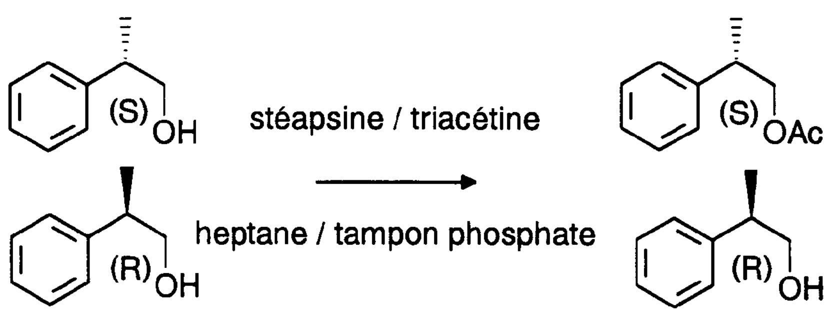


Dans un tube en verre de 4 ml, on charge 10 mg de Stéapsine (lipase issue du pancréas de porc) vendue par Biocatalyst et 0,4 ml d'un tampon phosphate de sodium de pH égal à 7 (100 mmol/l).

On additionne 10 mg de 2-phényl 1-propanol (0,075 mmol) dissous dans 0,5 ml d'heptane.

On mélange la phase aqueuse et la phase organique, par agitation magnétique.

On ajoute alors 100 µl (0,5 mmol) de triacétine.

On maintient le milieu réactionnel, sous agitation, à 25°C, pendant 1 heure 20 minutes.

On analyse la masse réactionnelle et l'on détermine le taux de transformation par chromatographie en phase gazeuse (colonne CARBOWAX® de 15 m) et l'on détermine l'excès énantiomérique de l'acétate formé par chromatographie

liquide en phase chirale (CHIRALPACK AD).

Les résultats obtenus sont les suivants :

TT = 32 %

ee [1-acétoxy 2-phénylpropane] = 69 %

E = 7,4

activité = 240 g/h.kg d'enzyme

Exemple 3 :

. Résolution du nortricyclanol

Le mélange est résolu par acétylation du (-) nortricyclanol selon la réaction suivante :

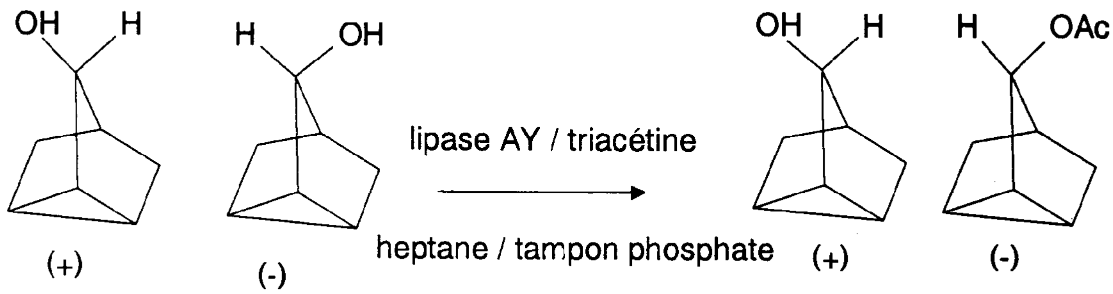


Dans un tube de 4 ml, on charge 0,5 g de lipase AY (provenant de *Candida cylindracea*) et 40 ml d'un tampon phosphate de sodium de pH égal à 7 (100 mmol/l).

On additionne 2,5 g de nortricyclanol (22,7 mmol) dissous dans 50 ml d'heptane.

On mélange la phase aqueuse et la phase organique, par agitation magnétique.

On ajoute alors 10 g (45 mmol) de triacétine.

Après 2 heures d'agitation à la température ambiante, on détermine le taux de transformation par chromatographie en phase gazeuse (colonne CARBOWAX ® de 15 m) et l'excès énantiomérique de l'acétate, par polarimétrie après séparation de l'alcool et l'acétate sur colonne de silice :

réf. $[\alpha]_D$ = - 47,4°

dans l'éther éthylique, référence définie par Yoshiki HIROSE et al [Chemistry Letters pp. 1939-1942(1989)].

Les résultats obtenus sont les suivants :

TT = 59 %

ee [acétate] = 66 %

E = 17

activité = 1475 g/h.kg d'enzyme

Exemple 4 :

. Résolution du menthol

Le mélange est résolu par acétylation du (-) menthol selon la réaction suivante :

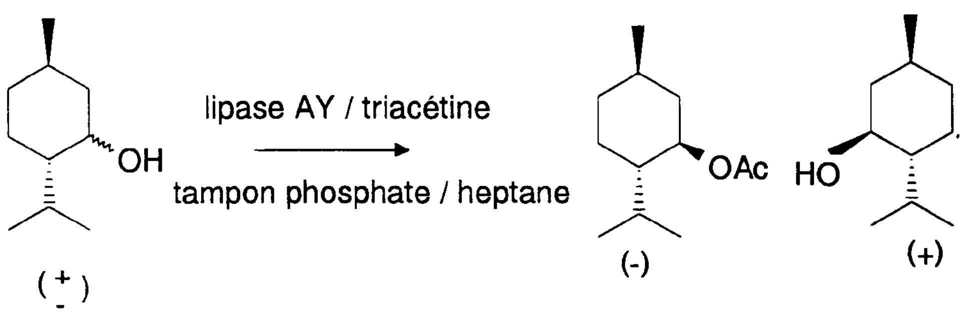


Dans un tube de 4 ml, on charge 10 mg de lipase AY et 0,4 ml d'un tampon phosphate de sodium de pH égal à 7 (250 mmol/l).

On additionne 10 mg de menthol (0,064 mmol) dissous dans 0,5 ml d'heptane.

On mélange la phase aqueuse et la phase organique, par agitation magnétique.

On ajoute alors 100 µl (0,5 mmol) de triacétine.

Après 2 heures 30 minutes d'agitation à la température ambiante, on détermine le taux de transformation par chromatographie en phase gazeuse (colonne CARBOWAX® de 15 m).

Les résultats obtenus sont les suivants :

TT = 47,7 %
activité = 191 g/h.kg d'enzyme

Exemple 5

. Résolution du menthol

Le mélange est résolu par acétylation du (-) menthol comme décrit dans l'exemple 4.

Dans un réacteur de 250 ml, on charge 1 g de lipase AY et 40 ml d'un tampon phosphate de sodium de pH égal à 7 (250 mmol/l).

On additionne 10 g de menthol (64 mmol) dissous dans 50 ml d'heptane.

On mélange la phase aqueuse et la phase organique, par agitation magnétique.

On ajoute alors 10 ml (50 mmol) de triacétine.

Après 120 heures d'agitation à température ambiante, on ajoute 1 g de lipase AY et 10 ml de triacétine.

Après 170 heures d'agitation à température ambiante, la réaction est stoppée.

On effectue la décantation par arrêt de l'agitation et la séparation des phases.

La phase aqueuse est extraite avec 50 ml d'heptane.

Les phases heptaniques sont réunies et le solvant évaporé.

On sépare l'alcool de l'acétate sur colonne de silice.

On détermine la pureté optique de l'acétate formé par rapport à un étalon commercial de (-) menthol par polarimètrie en procédant à une hydrolyse basique (KOH à 38 % + méthanol rapport V/V = 1/1).

Les résultats obtenus sont les suivants :

TT = 47,5 %
ee[acétate] = 91 %
E = 54

Exemple 6 :

. Résolution du 4-tert-butylcyclohexanol

Le mélange est résolu par acétylation de l'isomère équatorial selon la réaction suivante :

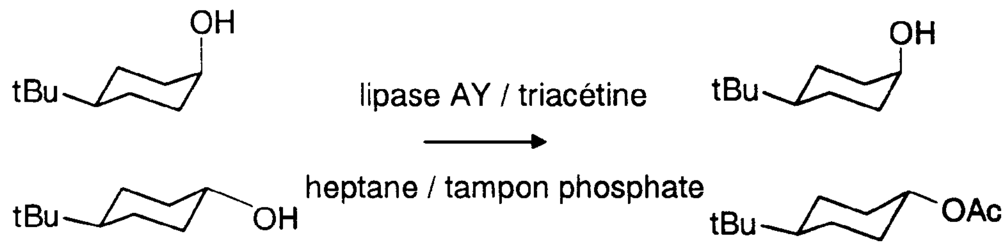


Il contient 70 % d'isomère équatorial et 30 % d'isomère axial.

Dans un réacteur de 6 litres, on met en solution 2 g de lipase AY dans 1,25 litre d'un tampon phosphate de sodium de pH égal à 7 (100 mmol/l).

On additionne 1,25 l d'heptane contenant 150 g de 4-tert-butylcyclohexanol (961 mmoles) contenant 70 % d'isomère équatorial pour 30 % d'isomère axial. La réaction est initiée par l'ajout de 200 ml de triacétine (975 mmoles).

Le réacteur est maintenu à température ambiante et agité mécaniquement de façon à assurer la microémulsion du système.

Le pH de la phase aqueuse est régulé à 7 par addition d'une solution aqueuse de soude 12 N.

Différents ajouts sont effectués au cours de la réaction :

- 0,2 g de lipase au bout de 70 heures ; 0,8 g de lipase au bout de 165 heures,
- 100 ml de triacétine au bout de 165 heures ; 100 ml de triacétine au bout de 189 heures.

Au bout de 261 heures, 70,4 % du 4-tert-butylcyclohexanol sont transformés.

La réaction est stoppée par décantation et séparation des phases.

La conversion du substrat et la sélectivité de la réaction sont mesurées par chromatographie en phase gazeuse.

TT = 70,4 %

La phase aqueuse est lavée à l'heptane, et les phases organiques sont séchées puis évaporées. 180 g de produits sont récupérés.

Après séparation sur colonne de silice, on obtient 130 g d'acétate de 4-tert-butylcyclohexyle équatorial et 39 g de 4-tert-butylcyclohexanol axial.

Les résultats obtenus sont les suivants :

ed alcool = 93 %

ed acétate = 97 %

Exemples 7 à 10 :

Essais a à d :

Résolution du 4-tert-butylcyclohexanol

Dans cette série d'exemples, on met en évidence l'influence de la nature de l'agent d'acétylation sur la transestérification du 4-tert-butylcyclohexanol catalysée par la lipase AY.

De plus, on montre l'influence du milieu bi-phasique heptane/eau (exemples 7 à 10) par rapport à un milieu anhydre heptane (essais a à d).

Exemples 7 à 10 :

Dans un tube de 4 ml, on charge 10 mg de lipase AY et 0,4 ml d'un tampon phosphate de sodium de pH égal à 7 (100 mmol/l).

On additionne 15,6 mg de 4-tert-butylcyclohexanol (0,1 mmol) dissous dans 0,5 ml d'heptane.

On mélange la phase aqueuse et la phase organique par agitation magnétique.

On ajoute alors 0,2 mmol d'un agent d'acylation dont la nature est précisée dans le tableau I.

Après une durée d'agitation précisée dans le tableau suivant, à 25°C, on détermine le taux de transformation et l'excès énantiomérique par chromatographie en phase gazeuse.

Les conditions et résultats sont répertoriés dans le tableau I.

Essais a à d :

Dans un tube de 4 ml, on charge 10 mg de lipase AY, 0,5 ml d'heptane et 15,6 mg de 4-tert-butylcyclohexanol (0,1 mmol).

On mélange la phase aqueuse et la phase organique, par agitation magnétique.

On ajoute alors 0,2 mmol d'un agent d'acylation dont la nature est précisée das le tableau I.

Après une durée d'agitation précisée dans le tableau suivant, à 25°C, on détermine le taux de transformation et l'excès énantiomérique par chromatographie en phase gazeuse.

Les conditions et résultats sont répertoriés dans le tableau I.

Tableau I

| Réf. ex. | Agent acylant | Milieu | Durée (h) | TT alcool (%) | ed acétate (%) | Activité (g/h.kg de lipase) |
|---|---|---|---|---|---|---|
| a | triacétine | heptane | 1,7 | 39 | 100 | 360 |
| 7 | | heptane/eau | 0,5 | 37 | 100 | 1100 |
| b | vinylacétate | heptane | 2,7 | 15 | 100 | 80 |
| 8 | | heptane/eau | | 31 | 91 | 170 |
| c | anhydride acétique | heptane | 2,7 | 18 | 100 | 100 |
| 9 | | heptane/eau | | 58 | 35 | 325 |

Suite du Tableau sur la page suivante

Tableau I (suite)

| Réf. ex. | Agent acylant | Milieu | Durée (h) | TT alcool (%) | ed acétate (%) | Activité (g/h.kg de lipase) |
|---|---|---|---|---|---|---|
| d | éthylacétate | heptane | 2,7 | 1 | 100 | 5 |
| 10 | | heptane/eau | | 3 | 100 | 17 |

Il ressort de l'examen de ce tableau que le système hydoorganique est cinétiquement plus favorable. Parmi les différents agents d'acylation, la triacétine reste le meilleur agent d'acétylation suivie par les esters activés.

Exemples 11 à 14 :

Dans cette série d'exemples, on montre l'Influence de la teneur en lipase AY sur l'acétylation du 4-tert-butylcyclohexanol par la triacétine.

Dans un tube de 4 ml, on charge x mg de lipase AY et 0,4 ml d'un tampon phosphate de sodium de pH égal à 7 (100 mmol/l).

On additionne 50 mg de 4-tert-butylcyclohexanol (0,32 mmol) dissous dans 0,5 ml d'heptane.

On mélange la phase aqueuse et la phase organique par agitation magnétique.

On ajoute alors 0,1 ml de triacétine soit 0,5 mmol.

Après une durée de réaction de 0,5 heure à 25°C, on détermine le taux de transformation et l'excès énantiomérique par chromatographie en phase gazeuse.

Les conditions et résultats sont répertoriés dans le tableau II.

Tableau II

| Réf. ex. | Lipase AY (g/l) | TT (%) | Activité (g/h.kg d'enzyme) | ed acétate (%) |
|---|---|---|---|---|
| 11 | 10 | 19 | 2000 | 99 |
| 12 | 5 | 12 | 2500 | 99 |
| 13 | 1 | 2,5 | 2800 | 99 |
| 14 | 0,5 | 1,7 | 3300 | 99 |

Exemples 15 à 19 :

Au cours de la réaction d'acétylation du 4-tert-butylcyclohexanol, on note une réaction parasite d'hydrolyse de la triacétine.

Dans cette série d'exemples, on montre l'influence du pH sur la compétition des réactions d'hydrolyse et de transestérification de la triacétine.

Une partie de la triacétine est consommée par réaction d'hydrolyse et il a été trouvé que le choix du pH permettait de minimiser l'hydrolyse.

A cet effet, on détermine le rendement en triacétine ($RT_{triacétine}$) par l'équation suivante :

$$RT_{triacétine} = \frac{\text{nb de moles de 4-tert-butylcyclohexanol estérifiées}}{\text{nb de moles de triacétine consommées}}$$

Cette détermination est effectuée à différents pH, en suivant le protocole opératoire suivant.

Dans un réacteur de 250 ml, on charge 50 mg de lipase AY, 30 ml de tampon phosphate de sodium de pH égal à 7 (5 mmol/l).

On additionne 1 g de 4-tert-butylcyclohexanol (6,4 mmol) dissous dans 50 ml d'heptane.

On mélange la phase aqueuse et la phase organique, par agitation magnétique.

On ajoute alors 20 ml (100 mmol) de triacétine.

Au cours de la réaction, le pH est régulé, selon les exemples, à une valeur allant de 5 à 9 par ajout d'une solution aqueuse de soude 2N, ce qui permet de déterminer la quantité de triacétine hydrolysée en fonction du pH.

On détermine le taux de transformation du 4-tert-butylcyclohexanol, par chromatographie en phase gazeuse.

Les résultats obtenus sont consignés dans le tableau III.

Tableau III

| Réf. ex. | pH | RT triacétine (%) |
|---|---|---|
| 15 | 5 | 41 |
| 16 | 6 | 36,5 |
| 17 | 7 | 34 |
| 18 | 8 | 26,3 |
| 19 | 9 | 11,9 |

L'examen du tableau III conduit à choisir un pH le plus bas possible afin d'éviter l'hydrolyse de la triacétine.

Exemples 20 à 23

Dans les exemples suivants, on met en évidence l'influence de la concentration du substrat de départ sur la compétition entre la réaction d'hydrolyse et de transestérification.

On reproduit le mode opératoire des exemples 15 à 19 à la différence près que l'on change la concentration du 4-tert-butylcyclohexanol, dans le milieu réactionnel.

Dans lesdits exemples, le pH est régulé à 7,0, par ajout de la solution aqueuse de soude 2N.

Les résultats obtenus sont consignés dans le tableau IV.

Tableau IV

| Réf. ex. | Concentration en 4 tert-butylcyclohexanol (g/l) | RT triacétine (%) |
|---|---|---|
| 20 | 0 | 0 |
| 21 | 1 | 4,1 |
| 22 | 10 | 32,2 |
| 23 | 100 | 36,3 |

Il ressort de l'examen du tableau IV qu'il est préférable d'avoir une concentration en substrat élevée afin de minimiser la réaction d'hydrolyse de la triacétine.

Exemples 24 à 30

On conduit les exemples 24 à 30 à différentes températures selon le mode opératoire défini ci-après.

Dans un tube de verre de 4 ml, on charge 20 mg de lipase AY et 0,9 ml d'un tampon phosphate de sodium de pH égal à 7 (100 mmol/l).

On additionne 50 mg de 4-tert-butylcyclohexanol (0,32 mmol) dissous dans 1,0 ml d'heptane.

On mélange la phase aqueuse et la phase organique, par agitation magnétique.

Par chauffage, on porte la masse réactionnelle à une température variant selon l'exemple et qui est précisée dans le tableau récapitulatif (tableau V).

La réaction est initiée par ajout de 100 µl (0,5 mmol) de triacétine.

Après 20 minutes de réaction, on analyse la masse réactionnelle par chromatographie en phase gazeuse.

Les résultats obtenus sont rassemblés dans le tableau V.

Tableau V

| Réf. ex. | Température (°C) | Activité (g/h.kg) |
|---|---|---|
| 24 | 20 | 970 |
| 25 | 30 | 1240 |
| 26 | 40 | 1820 |
| 27 | 45 | 2110 |

Suite du Tableau sur la page suivante

Tableau V (suite)

| Réf. ex. | Température (°C) | Activité (g/h.kg) |
|---|---|---|
| 28 | 50 | 2400 |
| 29 | 55 | 2600 |
| 30 | 60 | 2520 |

La température a une faible incidence sur la cinétique réactionnelle puisque l'on constate seulement un doublement de l'activité tous les 25°C.

Exemple 31

Le 4-tert-butylcyclohexanol axial obtenu selon l'exemple 6 est soumis à une acétylation selon une technique classique telle que par exemple, à l'aide du chlorure d'acétyle ou d'anhydride acétique.

On obtient l'acétate de 4-tert-butylcyclohexyle axial qui présente une odeur de pin fleuri.

Il est à noter que par rapport à un mélange cis/trans de l'acétate de 4-tert-butylcyclohexyle, l'odeur de la molécule acétate de 4-tert-butylcyclohexyle enrichie en isomère axial, est beaucoup plus intéressante que le mélange.

Exemple 32

L'acétate de 4-tert-butylcyclohexyle équatorial obtenu selon l'exemple 6 présente une note boisée verte.

Exemple 33

L'acétate d'hydratropyle (S) obtenu selon l'exemple 2 présente une note jonquille miellée.

**Revendications**

**1 -** Procédé de résolution d'un mélange d'alcools stéréoisomères caractérisé par le fait qu'il consiste :

- à faire réagir ledit mélange avec un agent d'acylation, en présence d'une hydrolase, en milieu bi-phasique, hydro-organique,
- à séparer l'isomère estérifié de l'isomère non estérifié afin d'obtenir d'une part, l'un des stéréoisomères de l'alcool et d'autre part, l'ester de l'autre stéréoisomère.

**2 -** Procédé selon la revendication 1 caractérisé par le fait que le mélange d'alcools stéréoisomères est un mélange d'énantiomères ou de stéréoisomères de position.

**3 -** Procédé selon l'une des revendications 1 et 2 caractérisé par le fait que le mélange d'alcools stéréoisomères est un mélange d'énantiomères d'un alcool répond à la formule (I) suivante :

$$R - OH \qquad (I)$$

dans ladite formule :

- R représente un radical hydrocarboné comprenant au moins 4 atomes, monovalent, substitué ou non, qui peut être un radical aliphatique, acyclique, saturé ou insaturé, linéaire ou ramifié ; un radical carbocyclique ou hétérocyclique, saturé ou insaturé, monocyclique ou polycyclique,
- le groupe hydroxyle pouvant être porté ou non par le carbone asymétrique.

**4** Procédé selon la revendication 3 caractérisé par le fait que l'alcool répond à la formule (I) dans laquelle R représente :

- un radical alkyle, alcényle, alcadiényle, alcynyle, linéaire ou ramifié ayant de préférence de 4 à 40 atomes de carbone : la chaîne hydrocarbonée pouvant être éventuellement interrompue par un hétéroatome (par exemple,

oxygène ou soufre) ou par l'un des groupes suivants : -CO-, -COO-, -OCOO-, $-SO_2-$ ,

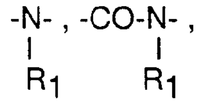

et/ou porteuse de l'un des substituants suivants :-OH, $-COOR_1$, -CHO, $-NO_2$, -X, $-CF_3$ : dans ces formules $R_1$ représente de préférence l'hydrogène ou un radical alkyle linéaire ou ramifié ayant de 1 à 4 atomes de carbone, de préférence, un radical méthyle ou éthyle,

- un radical aliphatique acyclique, saturé ou insaturé, linéaire ou ramifié éventuellement porteur d'un substituant cyclique : le reste aliphatique acyclique pouvant être relié au cycle par un lien valentiel ou par l'un des groupes suivants : -O-, -CO-, -COO-, -OCOO-, -S-, $-SO_2-$ ,

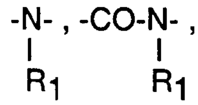

dans ces formules, $R_1$ ayant la signification donnée précédemment,

- un radical carbocyclique, monocyclique, saturé ou comprenant 1 ou 2 insaturations ; le nombre d'atomes de carbone dans le cycle pouvant varier de 3 à 8 atomes et étant égal de préférence à 5 ou 6,

- un radical hétérocyclique, monocyclique, saturé ou comprenant 1 ou 2 insaturations ; le nombre d'atomes dans le cycle pouvant varier de 3 à 8 atomes et étant égal de préférence à 5 ou 6,

- un radical carbocyclique, polycyclique, de préférence bicyclique, saturé ou comprenant 1 ou 2 insaturations ; le nombre d'atomes de carbone dans chaque cycle variant entre 3 et 6 et le nombre total d'atomes de carbone étant égal de préférence à 7.

- un radical hétérocyclique, polycyclique, de préférence bicyclique, saturé ou comprenant 1 ou 2 insaturations; le nombre d'atomes dans chaque cycle variant entre 3 et 6 et étant égal de préférence à 5 ou 6.

**5 -** Procédé selon l'une des revendications 3 et 4 caractérisé par le fait que l'alcool est un alcool aliphatique, saturé ou insaturé, linéaire ou ramifié, ayant de 4 à 30 atomes de carbone, pouvant éventuellement porté un substituant, par exemple, cyclique, et plus particulièrement, un groupe phényle, un atome d'halogène, un groupement amine ou alkoxy ayant généralement de 1 à 4 atomes de carbone.

**6 -** Procédé selon l'une des revendications 3 à 5 caractérisé par le fait que le mélange de stéréoisomères à séparer est un mélange de stéréoisomères du 2-phényl 1-propanol (ou alcool hydratropique), du 1-phényléthanol (ou alcool $\alpha$-phényléthylique) du menthol ou du nortricyclanol.

**7 -** Procédé selon l'une des revendications 1 à 6 caractérisé par le fait que le mélange d'alcools stéréoisomères est un mélange stéréoisomères d'alcools cycliques secondaires, de préférence du cyclohexanol, l'un présentant le groupe hydroxyle en position axiale et l'autre en position équatoriale; la conformation étant bloquée par la présence d'un groupement encombrant, de préférence un groupe alkyle aliphatique ou cycloaliphatique, ayant au moins 3 atomes de carbone jusqu'à 12 atomes de carbone.

**8 -** Procédé selon l'une des revendications 1 à 7 caractérisé par le fait que le mélange de stéréoisomères à séparer est un mélange de stéréoisomères de 4-tert-butylcyclohexanol, de 4-isobornylcyclohexanol, de 4-isocamphyl-cyclohexanol.

**9 -** Procédé selon l'une des revendications 1 à 8 caractérisé par le fait que l'agent d'acylation est soluble en phase aqueuse, et est de préférence, un ester d'acide organique ou à un anhydride d'acide organique.

**10 -** Procédé selon la revendication 9 caractérisé par le fait que l'agent d'acylation répond à la formule (II) :

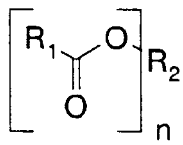

(II)

dans laquelle :

- n est un nombre égal à 1, 2 ou 3,
- $R_1$ représente un radical alkyle ayant une faible condensation en carbone,
- $R_2$ peut prendre les significations suivantes :
  . si n = 1, $R_2$ représente un radical alkyle ou alcényle de faible condensation en carbone ou un radical $-CO-R_3$, avec $R_3$, identique ou différent de $R_1$, ayant la même signification que $R_1$,
  . si n = 2, $R_2$ représente un radical alkylène ou alcénylène de faible condensation en carbone,
  . si n = 3, $R_2$ représente un radical alkyle triyle de faible condensation en carbone.

**11 -** Procédé selon l'une des revendications 9 et 10 caractérisé par le fait que l'agent d'acylation répond à la formule (II) dans laquelle :

- $R_1$ représente un radical alkyle ayant de 1 à 3 atomes de carbone,
- $R_2$ représente un radical méthyle ou vinyle, halogénométhyle, mono-, di- ou trihalogénométhyle, méthylsulfate, éthylsulfate pour n = 1 ; un radical diéthylène pour n = 2 ; un radical propanetriyle-1,2,3 pour n = 3 lorsque l'agent d'acylation est un ester d'acide organique.
- $R_1$ et $R_3$ sont identiques et représentent un radical alkyle ayant de 1 à 3 atomes de carbone, lorsque l'agent d'acylation est un anhydride d'acide.

**12 -** Procédé selon l'une des revendications 9 à 11 caractérisé par le fait que l'agent d'acylation est choisi parmi :

- la triacétine,
- l'acétate de vinyle,
- l'anhydride acétique.

**13 -** Procédé selon l'une des revendications 1 à 12 caractérisé par le fait que que l'enzyme est choisie parmi les hydrolases, et plus particulièrement les lipases, estérases ou acylases.

**14 -** Procédé selon la revendications 13 caractérisé par le fait que l'enzyme est une hydrolase appartenant à la classe EC.3 selon la nomenclature internationale et plus particulièrement aux carboxylestérases (3.1.1.1), aux triacylglycérol lipases (3.1.1.3) ou aux acylases (3.5.1.4).

**15 -** Procédé selon l'une des revendications 13 et 14 caractérisé par le fait que l'enzyme est une lipase issue d'organes (par exemple foies, pancréas ou glandes salivaires) d'organismes supérieurs comme de bovins (veaux) , porcs, chevaux, lapins ou provient de micro-organismes tels que, par exemple : *Géotricum candidum, Penicillium cyclopium, Penicillium roqueforti, Candida cylindracea*, *Rhizopus delemar, Rhizopus niveus, Mucor javanicus, Pseudomonas fluorescences, Pseudomonas species* ; une estérase de foie de porc ou de cheval et une acylase obtenue à partir des micro-organismes tels que *l'Aspergillus*.

**16 -** Procédé selon l'une des revendications 13 à 15 caractérisé par le fait que l'enzyme est obtenue à partir du *Candida cylindracea.*

**17 -** Procédé selon l'une des revendications 1 à 16 caractérisé par le fait que le pH de la phase aqueuse est choisi de telle sorte qu'il soit compatible avec l'activité de l'enzyme, de préférence, entre 5 et 8, et encore plus préférentiellement entre 5 et 7.

**18 -** Procédé selon la revendication 17 caractérisé par le fait que le pH est régulé par addition d'un agent de neutralisation, de préférence, la soude ou par ajout d'un agent tampon de préférence, phosphate, carbonate, bicarbonate, borate voire-même un agent tampon organique comme le tris-(hydroxyméthyl)amino-méthane.

**19 -** Procédé selon l'une des revendications 1 à 18 caractérisé par le fait que le solvant organique est un solvant organique non miscible à l'eau, de préférence, un solvant organique, aprotique, peu polaire.

**20 -** Procédé selon la revendication 19 caractérisé par le fait que le solvant organique est un hydrocarbure aliphatique ou aromatique, un hydrocarbure halogéné aliphatique ou aromatique, un éther-oxyde aliphatique, cycloaliphatique ou aromatique.

**21 -** Procédé selon l'une des revendications 1 à 20 caractérisé par le fait que le rapport volumique entre la phase aqueuse et la phase organique varie entre 0,1 et 10, de préférence entre 1,0 et 5,0.

**22 -** Procédé selon l'une des revendications 1 à 21 caractérisé par le fait que que la concentration du mélange d'alcools stéréoisomères dans le solvant organique est à une concentration élevée, de l'ordre de 1 mole/litre.

**23** Procédé selon l'une des revendications 1 à 22 caractérisé par le fait que la quantité de l'agent d'acylation est au moins égale à la quantité stoechiométrique, de préférence, en excès variant de 1 à 40 par rapport à la stoechiométrie, de préférence de 1 à 5.

**24 -** Procédé selon l'une des revendications 1 à 23 caractérisé par le fait que la quantité d'enzyme mise en oeuvre représente de 0,1 à 100 %, de préférence de 1 à 10 % du poids du mélange d'alcools stéréoisomères.

**25 -** Procédé selon l'une des revendications 1 à 24 caractérisé par le fait que la température de la réaction est choisie de préférence inférieure à 60°C, et plus préférentiellement entre 20 et 40°C.

**26 -** Procédé selon l'une des revendications 1 à 25 caractérisé par le fait que l'on prépare la phase aqueuse constituée du mélange d'eau, de l'enzyme et éventuellement l'agent permettant d'obtenir le bon pH, que l'on introduit le mélange d'alcools stéréoisomères à séparer dans le solvant organique ce qui compose la phase organique, que l'on procède au mélange des phases aqueuse et organique puis que l'on initie la réaction par ajout de l'agent d'acylation, que l'on porte le milieu réactionnel à la température désirée, tout en maintenant le milieu réactionnel sous bonne agitation, que l'on récupère en phase organique, l'un des stéréoisomères sous forme estérifiée et également l'autre stéréoisomère qui reste sous forme d'alcool.

**27 -** Alcools et alcools acylés, de préférence, acétylés, énantiomères ou stéréoisomères de position répondant à la formule (I) ou dérivés de la formule (I).

**28 -** Enantiomères (R) et (S) du 1-phényléthanol, énantiomères (R) et (S) du 1-phényléthylacétate.

**29 -** Enantiomères (R) et (S) de l'alcool hydratropique et énantiomères (R) et (S) de l'acétate d'hydratropyle.

**30 -** Nortricyclanol (+), nortricyclanol (-), nortricyclanol acétylé (+) et nortricyclanol acétylé (-).

**31 -** Menthol (+), menthol (-), acétate de menthyle (+) et acétate de menthyle (-).

**32 -** Stéréoisomères de position d'alcools ou d'alcools acylés cycliques secondaires, de préférence du cyclohexanol, l'un présentant un groupe hydroxyle ou ester (de préférence, acétate) en position axiale et l'autre, un groupe hydroxyle ou ester (de préférence, acétate), en position équatoriale : la conformation étant bloquée par la présence d'un groupement encombrant.

**33 -** Stéréoisomères d'alkylcyclohexanol ou d'alkylcyclohexanol acylé, de préférence, acétylé, présentant un groupe alkyle tel que tert-butyle, isobornyle et isocamphyle et se distinguant par la présence d'un groupe hydroxyle ou ester (de préférence, acétate), en position axiale ou équatoriale.

**34 -** Utilisation des alcools et des alcools acylés stéréoisomères selon l'une des revendications 27 à 33, dans le domaine de la parfumerie et de la pharmacie.

**35 -** Utilisation selon l'une des revendications 32 et 33 des stéréoisomères d'alkylcyclohexanol ou d'alkylcyclohexanol acylé, de préférence, acétylé dans le domaine de la parfumerie, et plus particulièrement de l'acétate de 4-tert-butylcyclohexyle axial.

**36 -** Procédé pour l'obtention de compositions parfumantes, de substances et produits parfumés destinés à la parfumerie caractérisé par le fait que l'on ajoute aux constituants usuels de ces compositions, substances et produits finis, une quantité efficace d'un alcool ou d'un alcool acylé stéréoisomère décrit dans l'une des revendications 27 à 33.

**37 -** Compositions parfumantes, substances et produits parfumés caractérisés par le fait qu'ils comprennent, à titre de principe actif ayant une influence sur l'odeur, une quantité efficace d'un alcool ou d'un alcool acylé stéréoisomère décrit dans l'une des revendications 27 à 33.

**38 -** Article parfumé selon la revendication 37 sous forme de parfum, d'eau de toilette, de lotions après rasage, de parfums, de savons, de gels de bain ou de douche, de produits déodorants ou antiperspirants, de shampooings ou tout produit capillaire, de talcs ou poudres de toute nature, de désodorisants d'air ambiant, de tout produit d'entretien ou de compositions détergentes, d'adoucissants pour textiles.

**39 -** Utilisation comme ingrédient parfumant, d'un alcool ou d'un alcool acylé, stéréoisomère décrit dans l'une des revendications 27 à 33.